# EUROPEAN PATENT APPLICATION

(11) **EP 4 567 018 A1**
(43) Date of publication of application: **11.06.2025**
(21) Application number: 23850079.7
(22) Date of filing: 01.08.2023
(51) Int. Cl.: C07C 17/02, B01J 31/02, C07C 19/08, C07C 22/04, C07C 25/02, C07C 29/62, C07C 31/38, C07C 45/63, C07C 49/80, C07C 49/813, C07C 67/307, C07C 69/65, C07D 213/89

(54) **METHOD FOR PRODUCING FLUORINATED ORGANIC COMPOUND**

(30) Priority: 02.08.2022 JP 2022123583
(71) Applicant: DAIKIN INDUSTRIES, LTD., Osaka-shi, Osaka 530-0001 (JP); Saga University, Saga-shi, Saga 840-8502 (JP)
(72) Inventor: HIGASHI, Masahiro, Osaka-Shi, Osaka 530-0001 (JP); KISHIKAWA, Yosuke, Osaka-Shi, Osaka 530-0001 (JP); KITAMURA, Tsugio, Saga-shi, Saga 840-8502 (JP); OYAMADA, Juzo, Saga-shi, Saga 840-8502 (JP)
(74) Representative: Hoffmann Eitle
(86) International application number: PCT/JP2023/028100
(87) International publication number: WO 2024/029525

(57) **Abstract**

Provided is a novel method for producing a fluorinated organic compound. This production method is for producing a fluorine adduct of a compound represented by the following formula (1): 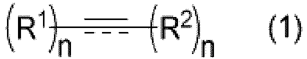 (wherein R¹, R², and n have the same meanings as the symbols described in the specification), the method comprising reacting the compound represented by formula (1) with
(A) at least one fluorine source selected from the group consisting of hydrogen fluoride, hydrogen fluoride salts, and fluoride salts;
(B) at least one iodine source selected from the group consisting of iodine, iodoaliphatic compounds, and iodoaromatic ring compounds; and
(C) at least one compound selected from the group consisting of compounds represented by the following formula (2-1) optionally having one or more substituents, and multimers thereof: 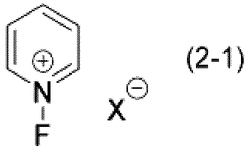 (wherein X is a conjugate base of a Brønsted acid), to add fluorine to the double bond or triple bond.

## Description

### Technical Field

The present disclosure relates to a method for producing a fluorinated organic compound.

### Background Art

Fluorinated organic compounds are extremely important compounds as various chemical products, such as functional materials, pharmaceutical and agrochemical compounds, and electronic materials, and as intermediates thereof.

As a method for producing a fluorinated organic compound, for example, NPL 1 proposes a method of difluorination of an olefin represented by the formula: CH₂=CH-CH₂-R, by reacting with p-iodotoluene (catalyst), Selectfluor (trademark) (1-chloromethyl-4-fluoro-1,4-diazoniabicyclo[2.2.2]octane bis(tetrafluoroborate)), and an HF source in a solvent, such as dichloroethane.

### Citation List

### Non-patent Literature

NPL 1: J. Am. Chem. Soc. 2016, 138, 5004-5007

### Summary of Invention

### Technical Problem

Selectfluor (trademark) in NPL 1 is expensive, and recovering Selectfluor after the reaction is difficult; therefore, there is room for improvement in terms of production costs.

The problem to be solved by the present disclosure is to provide a novel method for producing a fluorinated organic compound.

### Solution to Problem

The present disclosure encompasses the following embodiments.

### Item 1.

A method for producing a fluorine adduct of a compound represented by the following formula (1): 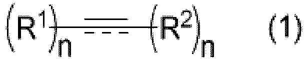 wherein
R¹ and R² are each independently a hydrogen atom or an organic group, or R¹ and R² optionally form a ring together with the two adjacent carbon atoms;
n is 1 or 2; and
the symbol 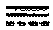 is a double bond or a triple bond,
with the proviso that
when the symbol is a triple bond, n is 1,
when the symbol is a double bond, n is 2,
two R¹s are optionally the same or different,
two R²s are optionally the same or different, or
two R¹s or two R²s optionally form a ring together with their adjacent carbon atom,
   the method comprising reacting the compound represented by formula (1) with
   (A) at least one fluorine source selected from the group consisting of hydrogen fluoride, hydrogen fluoride salts, and fluoride salts;
   (B) at least one iodine source selected from the group consisting of iodine, iodoaliphatic compounds, and iodoaromatic ring compounds; and
   (C) at least one compound selected from the group consisting of compounds represented by the following formula (2-1) optionally having one or more substituents, and multimers thereof: 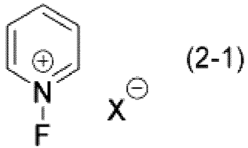 wherein
      X is a conjugate base of a Brønsted acid,
      to add fluorine to the double bond or triple bond.

### Item 2.

The production method according to Item 1, wherein the compound (C) is a compound represented by the following formula (2-2) optionally having one or more substituents: 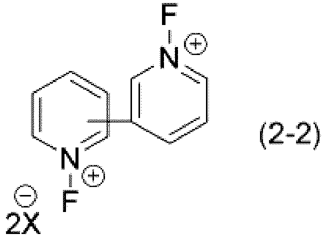 wherein
X is as defined above.

### Item 3.

The production method according to Item 1, wherein the compound (C) is a compound represented by the following formula (2-3) optionally having one or more substituents: 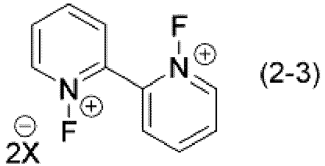 wherein
X is as defined above.

### Item 4.

The production method according to Item 3, wherein the compound (C) is a compound represented by formula (2-3).

### Item 5.

The production method according to any one of Items 1 to 4, wherein the compound (C) is used in an amount within the range of 0.1 to 10 moles per mole of the compound represented by formula (1).

### Item 6.

The production method according to any one of Items 1 to 4, wherein the compound (C) is used in an amount such that the amount of fluorine on nitrogen in the compound (C) is more than 1 mole per mole of the compound represented by formula (1).

### Item 7.

The production method according to any one of Items 1 to 4, wherein the fluorine source (A) is at least one member selected from the group consisting of hydrogen fluoride, hydrogen fluoride amine salts, alkali metal fluoride salts, and alkaline earth metal fluoride salts.

### Item 8.

The production method according to any one of Items 1 to 4, wherein the fluorine source (A) is used is an amount within the range of 0.1 to 1000 moles per mole of the compound represented by formula (1).

### Item 9.

The production method according to any one of Items 1 to 4, wherein the iodine source (B) is used in an amount within the range of 0.1 to 10 moles per mole of the compound represented by formula (1).

### Item 10.

The production method according to any one of Items 1 to 4, wherein the reaction is performed in the presence of a solvent.

### Item 11.

The production method according to any one of Items 1 to 4, wherein R¹ and R² are each independently a hydrogen atom, an alkyl group optionally having one or more substituents, a cycloalkyl group optionally having one or more substituents, an aryl group optionally having one or more substituents, an aralkyl group optionally having one or more substituents, a non-aromatic heterocyclic group optionally having one or more substituents, a cyano group, a formyl group, R^{A}O-, R^{A}CO-, R^{A}SO₂-, R^{A}COO-, (R^{A}) (R^{B})NCO-, R^{A}OCO-, R^{A}CONR^{B}-, R^{A}OSO₂-, or (R^{A})(R^{B})NSO₂-, wherein R^{A} and R^{B} are each independently an alkyl group optionally having one or more substituents, an aryl group optionally having one or more substituents, or an aralkyl group optionally having one or more substituents; or
R¹ and R² form a ring together with the two adjacent carbon atoms.

### Item 12.

The production method according to any one of Items 1 to 4, further comprising separating the compound (C) and/or a derivative thereof from a reaction mixture after the reaction.

### Item 13.

The production method according to any one of Items 1 to 4, further comprising reducing a reaction mixture after the reaction with a reducing agent.

### Item 14.

The production method according to Item 13, wherein the reducing agent is a sulfur-based reducing agent.

### Item 15.

A composition comprising at least one member selected from the group consisting of the following fluorine source (A) and iodine source (B), and comprising the following compound (C):
(A) at least one fluorine source selected from the group consisting of hydrogen fluoride, hydrogen fluoride salts, and fluoride salts;
(B) at least one iodine source selected from the group consisting of iodine, iodoaliphatic compounds, and iodoaromatic ring compounds; and
(C) at least one compound selected from the group consisting of compounds represented by the following formula (2-1) optionally having one or more substituents, and multimers thereof: 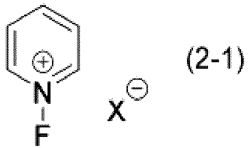 wherein
   X is a conjugate base of a Brønsted acid.

### Item 16.

A fluorinating agent comprising the composition according to Item 15.

### Advantageous Effects of Invention

The present disclosure provides a novel method for producing a fluorinated organic compound.

### Description of Embodiments

The above overview of the present disclosure is not intended to describe each of the disclosed embodiments or all of the implementations of the present disclosure.

The following description of the present disclosure more specifically exemplifies the embodiments of the examples.

In several parts of the present disclosure, guidance is provided through examples, and these examples can be used in various combinations.

In each case, the group of examples can function as a non-exclusive and representative group.

All publications, patents, and patent applications cited herein are incorporated herein by reference in their entirety.

### 1. Terms

Unless otherwise specified, the symbols and abbreviations in the present specification can be understood in the context of the present specification in the meanings commonly used in the technical field to which the present disclosure belongs.

In the present specification, the terms "comprise" and "contain" are used with the intention of including the terms "consisting essentially of" and "consisting of."

Unless otherwise specified, the steps, treatments, or operations described in the present specification can be performed at room temperature.

In the present specification, room temperature can refer to a temperature within the range of 10 to 40°C.

In the present specification, the phrase "Cₙ₋Cₘ" (n and m are each an integer of 1 or more, and n<m) indicates that the number of carbon atoms is n or more and m or less, as can be generally understood by a person skilled in the art.

In the present specification, the phrase "compound represented by formula (N)" (N is an integer of 1 or more) can be referred to as "compound (N)."

In the present specification, unless otherwise specified, examples of the halogen atom may include fluorine, chlorine, bromine, and iodine.

In the present specification, the "organic group" refers to a group containing one or more carbon atoms.

Examples of the organic group may include an alkyl group optionally having one or more substituents, an alkenyl group optionally having one or more substituents, an alkynyl group optionally having one or more substituents, a cycloalkyl group optionally having one or more substituents, a cycloalkenyl group optionally having one or more substituents, a cycloalkadienyl group optionally having one or more substituents, an aryl group optionally having one or more substituents, an aralkyl group optionally having one or more substituents, a non-aromatic heterocyclic group optionally having one or more substituents, a heteroaryl group optionally having one or more substituents, a cyano group, an aldehyde group, a carboxyl group, R^{R}O-, R^{R}CO-, R^{R}COO-, R^{R}SO₂-, R^{R}OCO-, and R^{R}OSO₂-(in these formulas, R^{R} is independently an alkyl group optionally having one or more substituents, an alkenyl group optionally having one or more substituents, an alkynyl group optionally having one or more substituents, a cycloalkyl group optionally having one or more substituents, a cycloalkenyl group optionally having one or more substituents, a cycloalkadienyl group optionally having one or more substituents, an aryl group optionally having one or more substituents, an aralkyl group optionally having one or more substituents, a non-aromatic heterocyclic group optionally having one or more substituents, or a heteroaryl group optionally having one or more substituents).

In the present specification, unless otherwise specified, examples of the hydrocarbon group may include an alkyl group, an alkenyl group, an alkynyl group, a cycloalkyl group, a cycloalkenyl group, a cycloalkadienyl group, an aryl group, an aralkyl group, and a group that is a combination of these groups.

In the present specification, unless otherwise specified, examples of the alkyl group may include linear or branched C₁₋C₁₆ alkyl groups (e.g., C₁-C₁₄ alkyl groups and C₁-C₁₂ alkyl groups), such as methyl, ethyl, propyl, isopropyl, butyl, isobutyl, sec-butyl, tert-butyl, pentyl, isopentyl, neopentyl, hexyl, heptyl, octyl, nonyl, and decyl.

In the present specification, unless otherwise specified, examples of the alkenyl group may include linear or branched C₂-C₁₀ alkenyl groups, such as vinyl, 1-propen-1-yl, 2-propen-1-yl, isopropenyl, 2-buten-1-yl, 4-penten-1-yl, and 5-hexen-1-yl.

In the present specification, unless otherwise specified, examples of the alkynyl group may include linear or branched C₂-C₁₀ alkynyl groups, such as ethynyl, 1-propyn-1-yl, 2-propyn-1-yl, 4-pentyn-1-yl, and 5-hexyn-1-yl.

In the present specification, unless otherwise specified, examples of the cycloalkyl group may include C₃-C₇ cycloalkyl groups, such as cyclopropyl, cyclobutyl, cyclopentyl, cyclohexyl, and cycloheptyl.

In the present specification, unless otherwise specified, examples of the cycloalkenyl group may include C₃-C₇ cycloalkenyl groups, such as cyclopropenyl, cyclobutenyl, cyclopentenyl, cyclohexenyl, and cycloheptenyl.

In the present specification, unless otherwise specified, examples of the cycloalkadienyl group may include C₄-C₁₀ cycloalkadienyl groups, such as cyclobutadienyl, cyclopentadienyl, cyclohexadienyl, cycloheptadienyl, cyclooctadienyl, cyclononadienyl, and cyclodecadienyl.

In the present specification, unless otherwise specified, the aryl group may be monocyclic, bicyclic, tricyclic, or tetracyclic.

In the present specification, unless otherwise specified, the aryl group may be a C₆-C₁₈ aryl group.

In the present specification, unless otherwise specified, examples of the aryl group may include phenyl, 1-naphthyl, 2-naphthyl, 2-biphenyl, 3-biphenyl, 4-biphenyl, and 2-anthryl.

In the present specification, unless otherwise specified, examples of the aralkyl group may include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, and 4-biphenylylmethyl.

In the present specification, unless otherwise specified, the non-aromatic heterocyclic group may be monocyclic, bicyclic, tricyclic, or tetracyclic.

In the present specification, unless otherwise specified, the non-aromatic heterocyclic group may be, for example, a non-aromatic heterocyclic group containing, in addition to carbon, 1 to 4 heteroatoms selected from oxygen, sulfur, and nitrogen as a ring-constituting atom.

In the present specification, unless otherwise specified, the non-aromatic heterocyclic group may be saturated or unsaturated.

In the present specification, unless otherwise specified, examples of the non-aromatic heterocyclic group may include tetrahydrofuryl, oxazolidinyl, imidazolinyl (e.g., 1-imidazolinyl, 2-imidazolinyl, and 4-imidazolinyl), aziridinyl (e.g., 1-aziridinyl and 2-aziridinyl), azetidinyl (e.g., 1-azetidinyl and 2-azetidinyl), pyrrolidinyl (e.g., 1-pyrrolidinyl, 2-pyrrolidinyl, and 3-pyrrolidinyl), piperidinyl (e.g., 1-piperidinyl, 2-piperidinyl, and 3-piperidinyl), azepanyl (e.g., 1-azepanyl, 2-azepanyl, 3-azepanyl, and 4-azepanyl), azocanyl (e.g., 1-azocanyl, 2-azocanyl, 3-azocanyl, and 4-azocanyl), piperazinyl (e.g., 1,4-piperazin-1-yl and 1,4-piperazin-2-yl), diazepinyl (e.g., 1,4-diazepin-1-yl, 1,4-diazepin-2-yl, 1,4-diazepin-5-yl, and 1,4-diazepin-6-yl), diazocanyl (e.g., 1,4-diazocan-1-yl, 1,4-diazocan-2-yl, 1,4-diazocan-5-yl, 1,4-diazocan-6-yl, 1,5-diazocan-1-yl, 1,5-diazocan-2-yl, and 1,5-diazocan-3-yl), tetrahydropyranyl (e.g., tetrahydropyran-4-yl), morpholinyl (e.g., 4-morpholinyl), thiomorpholinyl (e.g., 4-thiomorpholinyl), 2-oxazolidinyl, dihydrofuryl, dihydropyranyl, and dihydroquinolyl.

In the present specification, unless otherwise specified, examples of the heteroaryl group may include monocyclic aromatic heterocyclic groups (e.g., 5- or 6-membered monocyclic aromatic heterocyclic groups) and aromatic condensed heterocyclic groups (e.g., 5- to 18-membered aromatic condensed heterocyclic groups).

In the present specification, unless otherwise specified, examples of the 5- or 6-membered monocyclic aromatic heterocyclic groups may include pyrrolyl (e.g., 1-pyrrolyl, 2-pyrrolyl, and 3-pyrrolyl), furyl (e.g., 2-furyl and 3-furyl), thienyl (e.g., 2-thienyl and 3-thienyl), pyrazolyl (e.g., 1-pyrazolyl, 3-pyrazolyl, and 4-pyrazolyl), imidazolyl (e.g., 1-imidazolyl, 2-imidazolyl, and 4-imidazolyl), isoxazolyl (e.g., 3-isoxazolyl, 4-isoxazolyl, and 5-isoxazolyl), oxazolyl (e.g., 2-oxazolyl, 4-oxazolyl, and 5-oxazolyl), isothiazolyl (e.g., 3-isothiazolyl, 4-isothiazolyl, and 5-isothiazolyl), thiazolyl (e.g., 2-thiazolyl, 4-thiazolyl, and 5-thiazolyl), triazolyl (e.g., 1,2,3-triazol-4-yl and 1,2,4-triazol-3-yl), oxadiazolyl (e.g., 1,2,4-oxadiazol-3-yl and 1,2,4-oxadiazol-5-yl), thiadiazolyl (e.g., 1,2,4-thiadiazol-3-yl and 1,2,4-thiadiazol-5-yl), tetrazolyl, pyridyl (e.g., 2-pyridyl, 3-pyridyl, and 4-pyridyl), pyridazinyl (e.g., 3-pyridazinyl and 4-pyridazinyl), pyrimidinyl (e.g., 2-pyrimidinyl, 4-pyrimidinyl, and 5-pyrimidinyl), and pyrazinyl.

In the present specification, unless otherwise specified, examples of the 5- to 18-membered aromatic condensed heterocyclic groups may include isoindolyl (e.g., 1-isoindolyl, 2-isoindolyl, 3-isoindolyl, 4-isoindolyl, 5-isoindolyl, 6-isoindolyl, and 7-isoindolyl), indolyl (e.g., 1-indolyl, 2-indolyl, 3-indolyl, 4-indolyl, 5-indolyl, 6-indolyl, and 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, and 7-benzo[b]furanyl), benzo[c]furanyl (e.g., 1-benzo[c]furanyl, 4-benzo[c]furanyl, and 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, and 7-benzo[b]thienyl), benzo[c]thienyl (e.g., 1-benzo[c]thienyl, 4-benzo[c]thienyl, and 5-benzo[c]thienyl), indazolyl (e.g., 1-indazolyl, 2-indazolyl, 3-indazolyl, 4-indazolyl, 5-indazolyl, 6-indazolyl, and 7-indazolyl), benzimidazolyl (e.g., 1-benzimidazolyl, 2-benzimidazolyl, 4-benzimidazolyl, and 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, and 1,2-benzisoxazol-7-yl), benzoxazolyl (e.g., 2-benzoxazolyl, 4-benzoxazolyl, 5-benzoxazolyl, 6-benzoxazolyl, and 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, and 1,2-benzisothiazol-7-yl), benzothiazolyl (e.g., 2-benzothiazolyl, 4-benzothiazolyl, 5-benzothiazolyl, 6-benzothiazolyl, and 7-benzothiazolyl), isoquinolyl (e.g., 1-isoquinolyl, 3-isoquinolyl, 4-isoquinolyl, and 5-isoquinolyl), quinolyl (e.g., 2-quinolyl, 3-quinolyl, 4-quinolyl, 5-quinolyl, and 8-quinolyl), cinnolinyl (e.g., 3-cinnolinyl, 4-cinnolinyl, 5-cinnolinyl, 6-cinnolinyl, 7-cinnolinyl, and 8-cinnolinyl), phthalazinyl (e.g., 1-phthalazinyl, 4-phthalazinyl, 5-phthalazinyl, 6-phthalazinyl, 7-phthalazinyl, and 8-phthalazinyl), quinazolinyl (e.g., 2-quinazolinyl, 4-quinazolinyl, 5-quinazolinyl, 6-quinazolinyl, 7-quinazolinyl, and 8-quinazolinyl), quinoxalinyl (e.g., 2-quinoxalinyl, 3-quinoxalinyl, 5-quinoxalinyl, 6-quinoxalinyl, 7-quinoxalinyl, and 8-quinoxalinyl), pyrazolo[1,5-a]pyridyl (e.g., pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, and pyrazolo[1,5-a]pyridin-7-yl), imidazo[1,2-a]pyridyl (e.g., imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, and imidazo[1,2-a]pyridin-8-yl).

In the present specification, examples of R^{R}O- may include alkoxy (e.g., C₁-C₁₀ alkoxy, such as methoxy, ethoxy, propoxy, and butoxy), cycloalkoxy (e.g., C₃-C₇ cycloalkoxy, such as cyclopentoxy and cyclohexoxy), aryloxy (e.g., C₆-C₁₈ aryloxy, such as phenoxy and naphthoxy), and aralkyloxy (e.g., C₇-C₁₉ aralkyloxy, such as benzyloxy and phenethyloxy).

In the present specification, examples of R^{R}CO- may include alkylcarbonyl (e.g., (C₁-C₁₀ alkyl)carbonyl, such as acetyl, propionyl, and butyryl), cycloalkylcarbonyl (e.g., (C₃-C₇ cycloalkyl)carbonyl, such as cyclopentanoyl and cyclohexanoyl), arylcarbonyl (e.g., (C₆-C₁₈ aryl)carbonyl, such as benzoyl and naphthoyl), and aralkylcarbonyl (e.g., (C₇-C₁₉ aralkyl)carbonyl, such as benzylcarbonyl and phenethylcarbonyl).

In the present specification, examples of R^{R}COO- may include alkylcarbonyloxy (e.g., (C₁-C₁₀ alkyl)carbonyloxy, such as acetyloxy, propionyloxy, and butyryloxy), cycloalkylcarbonyloxy (e.g., (C₃-C₇ cycloalkyl)carbonyloxy, such as cyclopentanoyloxy and cyclohexanoyloxy), arylcarbonyloxy (e.g., (C₆-C₁₈ aryl)carbonyloxy, such as benzoyloxy and naphthoyloxy), and aralkylcarbonyloxy (e.g., (C₇-C₁₉ aralkyl)carbonyloxy, such as benzylcarbonyloxy and phenethylcarbonyloxy).

In the present specification, examples of R^{R}SO₂- may include alkylsulfonyl (e.g., C₁-C₁₀ alkylsulfonyl, such as methylsulfonyl, ethylsulfonyl, and propylsulfonyl), cycloalkylsulfonyl (e.g., C₄-C₈ cycloalkylsulfonyl, such as cyclopentylsulfonyl and cyclohexylsulfonyl), arylsulfonyl (e.g., C₆-C₁₈ arylsulfonyl, such as phenylsulfonyl and naphthylsulfonyl), and aralkylsulfonyl (e.g., C₇-C₁₉ aralkylsulfonyl, such as benzylsulfonyl and phenethylsulfonyl).

In the present specification, examples of R^{R}OCO- may include alkoxycarbonyl (e.g., C₁-C₁₀ alkoxy)carbonyl, such as methoxycarbonyl, ethoxycarbonyl, and propoxycarbonyl), cycloalkoxycarbonyl (e.g., (C₃-C₇ cycloalkoxy)carbonyl, such as cyclopentoxycarbonyl and cyclohexoxycarbonyl), aryloxycarbonyl (e.g., (C₆-C₁₈ aryloxy)carbonyl, such as phenoxycarbonyl and naphthoxycarbonyl), and aralkyloxycarbonyl (e.g., (C₇-C₁₉ aralkyloxy)carbonyl, such as benzyloxycarbonyl and phenethyloxycarbonyl.

In the present specification, examples of R^{R}OSO₂- may include alkoxysulfonyl (e.g., C₁-C₁₀ alkoxysulfonyl, such as methoxysulfonyl, ethoxysulfonyl, and propoxysulfonyl), cycloalkoxysulfonyl (e.g., C₃-C₇ cycloalkoxysulfonyl, such as cyclopentoxysulfonyl and cyclohexoxysulfonyl), aryloxysulfonyl (e.g., C₆-C₁₈ aryloxysulfonyl, such as phenoxysulfonyl and naphthoxysulfonyl), and aralkyloxysulfonyl (e.g., C₇-C₁₉ aralkyloxysulfonyl, such as benzyloxysulfonyl and phenethyloxysulfonyl).

In the present specification, examples of the substituents in the "hydrocarbon group optionally having one or more substituents," "alkyl group optionally having one or more substituents," "alkenyl group optionally having one or more substituents," "alkynyl group optionally having one or more substituents," "cycloalkyl group optionally having one or more substituents," "cycloalkenyl group optionally having one or more substituents," "cycloalkadienyl group optionally having one or more substituents," "aryl group optionally having one or more substituents," "aralkyl group optionally having one or more substituents," "non-aromatic heterocyclic group optionally having one or more substituents," and "heteroaryl group optionally having one or more substituents" may include a halo group, a nitro group, a cyano group, an oxo group, a thioxo group, a carboxyl group, a sulfo group, a sulfamoyl group, a sulfinamoyl group, a sulfenamoyl group, R^{R}O-, R^{R}CO-, R^{R}COO-, R^{R}SO₂-, R^{R}OCO-, and R^{R}OSO₂- (in these formulas, R^{R} is as defined above) .

Of these substituents, examples of the halo group may include fluoro, chloro, bromo, and iodo.

The number of substituents may be within the range of 1 to the maximum substitutable number (e.g., 1, 2, 3, 4, 5, or 6).

In the present specification, the term "electron-withdrawing group" refers to a group having a positive op value according to Hammett's rule.

### 2. Method for Producing a Fluorine Adduct of Compound (1)

The method for producing a fluorine adduct of compound (1) comprises step A of reacting compound (1) with fluorine source (A), iodine source (B), and compound (C) to add fluorine (e.g., monofluorine or difluorine) to the double bond or triple bond of compound (1).

### 2-1. Compound (1)

In one embodiment of compound (1), R¹ and R² are preferably each independently a hydrogen atom, an alkyl group optionally having one or more substituents, a cycloalkyl group optionally having one or more substituents, an aryl group optionally having one or more substituents, an aralkyl group optionally having one or more substituents, a non-aromatic heterocyclic group optionally having one or more substituents, a cyano group, a formyl group, R^{A}O-, R^{A}CO-, R^{A}SO₂-, R^{A}COO-, (R^{A})(R^{B})NCO-, R^{A}OCO-, R^{A}CONR^{B}-, R^{A}OSO₂-, or (R^{A})(R^{B})NSO₂-, wherein R^{A} and R^{B} are each independently an alkyl group optionally having one or more substituents, an aryl group optionally having one or more substituents, or an aralkyl group optionally having one or more substituents.

The substituents may be any group having a structure that enables substitution with respect to the target and may be, for example, at least one member selected from the group consisting of halo groups, a hydroxy group, alkoxy groups, alkylcarbonyl groups, alkylcarbonyloxy groups, alkoxycarbonyl groups, arylcarbonyloxy groups, aryloxycarbonyl groups, and aryl groups. The number of substituents may be within the range of 1 to the maximum substitutable number (e.g., 1, 2, or 3).

In the above embodiment, at least one of R¹ and R² may be an electron-withdrawing group, such as a cyano group, a formyl group, R^{A}CO-, R^{A}SO₂-, R^{A}OCO-, or R^{A}OSO₂-. The reaction also proceeds with such compound (1) having an electron-deficient double bond or triple bond.

In another embodiment of compound (1), R¹ and R² preferably form a ring together with the two adjacent carbon atoms. The ring is usually formed when the symbol is a double bond and n is 2. Examples of the ring include cycloalkene rings corresponding to the groups mentioned above as examples of the "cycloalkenyl group," and non-aromatic heterocyclic rings corresponding to the groups with carbon-carbon double bond(s) among the groups mentioned above as examples of the "non-aromatic heterocyclic group."

The ring optionally has one or more substituents.

Examples of the substituents may include a hydrocarbon group, a halo group, a nitro group, a cyano group, an oxo group, a thioxo group, a carboxyl group, a sulfo group, a sulfamoyl group, a sulfinamoyl group, a sulfenamoyl group, RRO-, RRCO-, RRCOO-, RRSO₂-, RROCO-, and RROSO₂- (in these formulas, RR is as defined above).

The number of substituents may be within the range of 1 to the maximum substitutable number (e.g., 1, 2, 3, 4, or 5).

Preferable examples of compound (1) include a compound represented by the following formula (1A): 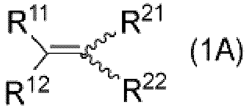 wherein
R¹¹, R¹², R²¹, and R²² are each independently a hydrogen atom or an organic group, R¹¹ and R¹², or R²¹ and R²² form a ring together with the one adjacent carbon atom, or R¹¹ and R²¹, R¹¹ and R²², R¹² and R²¹, or R¹² and R²² form a ring together with the two adjacent carbon atoms;
the symbol 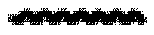 indicates a *cis* configuration or a *trans* configuration; and
a compound represented by the following formula (1B): 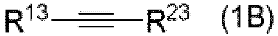 wherein
   R¹³ and R²³ are each independently a hydrogen atom or an organic group.

In one embodiment of compound (1A), R¹¹, R¹², R²¹, and R²² are preferably each independently a hydrogen atom, an alkyl group optionally having one or more substituents, a cycloalkyl group optionally having one or more substituents, an aryl group optionally having one or more substituents, an aralkyl group optionally having one or more substituents, a non-aromatic heterocyclic group optionally having one or more substituents, a cyano group, a formyl group, R^{A}O-, R^{A}CO-, R^{A}SO₂-, R^{A}COO-, (R^{A})(R^{B})NCO-, R^{A}OCO-, R^{A}CONR^{B}-, R^{A}OSO₂-, or (R^{A})(R^{B})NSO₂-, wherein R^{A} and R^{B} are each independently an alkyl group optionally having one or more substituents, an aryl group optionally having one or more substituents, or an aralkyl group optionally having one or more substituents.

In the above embodiment, it is preferable that R²² is a hydrogen atom, and it is more preferable that R¹² and R²² are hydrogen atoms. It is also preferable that R¹², R²¹, and R²² are hydrogen atoms.

In the above embodiment, it is also preferable that R¹² and R²² are hydrogen atoms and R¹¹ and R²¹ are not hydrogen atoms. The reaction also proceeds smoothly with such compound (1) having a double bond inside. At least one of R¹¹ and R²¹ may be an electron-withdrawing group, such as a cyano group, a formyl group, R^{A}CO-, R^{A}SO₂-, R^{A}OCO-, or R^{A}OSO₂-. The reaction also proceeds with such compound (1) having an electron-deficient double bond.

Preferable examples of compound (1A) include compounds represented by the following formulas (1a) to (1d): 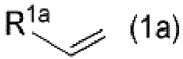 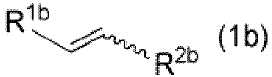 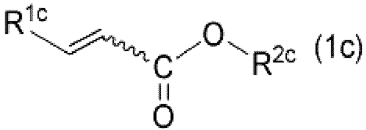 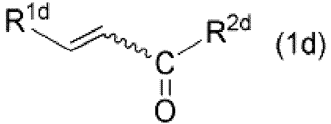 wherein
R^{1a} is an alkyl group optionally having one or more substituents, an aryl group optionally having one or more substituents, or an aralkyl group optionally having one or more substituents,
R^{1b} and R^{2b} are each independently an alkyl group optionally having one or more substituents, an aryl group optionally having one or more substituents, or an aralkyl group optionally having one or more substituents,
R^{1c} is a hydrogen atom, an alkyl group optionally having one or more substituents, an aryl group optionally having one or more substituents, or an aralkyl group optionally having one or more substituents,
R^{2c} is an alkyl group optionally having one or more substituents, R^{1d} is a hydrogen atom, an alkyl group optionally having one or more substituents, an aryl group optionally having one or more substituents, or an aralkyl group optionally having one or more substituents, and
R^{2d} is an alkyl group optionally having one or more substituents, an aryl group optionally having one or more substituents, or an aralkyl group optionally having one or more substituents. The substituents may be any group having a structure that enables substitution with respect to the target and may be, for example, at least one member selected from the group consisting of halo groups, a hydroxyl group, alkoxy groups, alkylcarbonyl groups, alkylcarbonyloxy groups, alkoxycarbonyl groups, arylcarbonyloxy groups, aryloxycarbonyl groups, and aryl groups. The number of substituents may be within the range of 1 to the maximum substitutable number (e.g., 1, 2, or 3).

In one embodiment of compound (1a), R^{1a} is an alkyl group optionally having one or more substituents (e.g., a halo group, a hydroxy group, or an alkoxycarbonyl group), or an aralkyl group optionally having one or more substituents (e.g., a halo group, a hydroxy group, or an alkoxycarbonyl group).

In one embodiment of compound (1c), R^{1c} is an aryl group optionally having one or more substituents (e.g., a halo group).

In one embodiment of compound (1d), R^{1d} is an aryl group optionally having one or more substituents (e.g., a halo group), and R^{2d} is an alkyl group optionally having one or more substituents (e.g., a halo group), or an aryl group optionally having one or more substituents (e.g., a halo group).

In one embodiment of compound (1B), R¹³ and R²³ are preferably each independently a hydrogen atom, an alkyl group optionally having one or more substituents, a cycloalkyl group optionally having one or more substituents, an aryl group optionally having one or more substituents, an aralkyl group optionally having one or more substituents, a non-aromatic heterocyclic group optionally having one or more substituents, a cyano group, a formyl group, R^{A}O-, R^{A}CO-, R^{A}SO₂-, R^{A}COO-, (R^{A})(R^{B})NCO-, R^{A}OCO-, R^{A}CONR^{B}-, R^{A}OSO₂-, or (R^{A})(R^{B})NSO₂-, wherein R^{A} and R^{B} are each independently an alkyl group optionally having one or more substituents, an aryl group optionally having one or more substituents, or an aralkyl group optionally having one or more substituents.

In the above embodiment, it is also preferable that R¹³ and R²³ are not hydrogen atoms. The reaction also proceeds smoothly with such compound (1) having a triple bond inside. Further, at least one of R¹³ and R²³ may be an electron-withdrawing group, such as a cyano group, a formyl group, R^{A}CO-, R^{A}SO₂-, R^{A}OCO-, or R^{A}OSO₂-. The reaction also proceeds with such compound (1) having an electron-deficient triple bond.

### 2-2. Fluorine Source (A)

Of fluorine sources (A), hydrogen fluoride may be used as an aqueous solution (hydrofluoric acid). The aqueous solution may be, for example, an aqueous solution having a hydrogen fluoride concentration of 10 to 70 mass%.

Of fluorine sources (A), examples of hydrogen fluoride salts include hydrogen fluoride amine salts and hydrogen fluoride ammonium salts.

In the hydrogen fluoride amine salts, the amine may be a chain amine or a cyclic amine.

Examples of chain amines include aliphatic primary amines, aliphatic secondary amines, and aliphatic tertiary amines.

Examples of aliphatic primary amines include C₁-C₆ alkylamines, such as methylamine, ethylamine, propylamine, butylamine, pentylamine, and hexylamine.

Examples of aliphatic secondary amines include di-C₁-C₆ alkylamines, such as dimethylamine, diethylamine, dipropylamine, dibutylamine, dipentylamine, and dihexylamine.

Examples of aliphatic tertiary amines include tri-C₁-C₆ alkylamines, such as trimethylamine, triethylamine, diisopropylethylamine, tributylamine, and N,N,N',N'-tetramethylethylenediamine.

Examples of cyclic amines include aliphatic cyclic amines and aromatic cyclic amines.

Examples of aliphatic cyclic amines include piperidine, piperazine, pyrrolidine, morpholine, N-methylpiperazine, N-methylpyrrolidine, 5-diazabicyclo[4.3.0]non-5-ene, and 1,4-diazabicyclo[2.2.2]octane.

Examples of aromatic cyclic amines include pyridine, pyrimidine, pyrazine, quinoline, and imidazole.

Preferable examples of hydrogen fluoride amine salts include hydrogen fluoride triethylamine salts (Et₃N•xHF, wherein x is an integer of 1 or more, preferably 1 to 10, and more preferably 3 to 7), hydrogen fluoride pyridine salts (Py•xHF, wherein x is an integer of 1 or more, and preferably 1 to 10), such as Olah's reagent, and combinations thereof.

Examples of hydrogen fluoride ammonium salts include a compound represented by the following formula (A1):

NQ₄F•xHF (A1)

wherein Q is each independently a hydrogen atom, an alkyl group, a cycloalkyl group, an aryl group, or an aralkyl group, or two or three Qs are bonded together to form a ring, and x is an integer of 1 or more.

In one embodiment of compound (A1), Q is preferably an alkyl group, and more preferably a C₁-C₆ alkyl group. X is preferably 1 to 10.

Preferable examples of compound (A1) include ammonium hydrogen fluoride (NH₄F•HF), hydrogen fluoride-tetramethylammonium fluoride, hydrogen fluoride-tetraethylammonium fluoride, hydrogen fluoride-tetrapropylammonium fluoride, and hydrogen fluoride-tetrabutylammonium fluoride.

Of fluoride sources (A), example of fluoride salts include a compound represented by the following formula (A2):

M¹Fₘ₁ (A2)

wherein M¹ is an alkali metal or an alkaline earth metal; and m1 is 1 or 2, depending on the valence of M¹.

In one embodiment of compound (A2), M¹ is preferably Li, Na, K, Ca, or Cs, M¹ is more preferably Na, K, or Ca, and M¹ is still more preferably K.

The fluoride salts may be preferably alkali metal fluoride salts (compounds (A2) in which M¹ is an alkali metal, and m1 is 1) or alkaline earth metal fluoride salts (compounds (A2) in which M¹ is an alkaline earth metal, and m1 is 2).

Fluorine sources (A) may be used singly or in a combination of two or more. In a preferable embodiment, fluorine source (A) is at least one member selected from the group consisting of hydrogen fluoride, hydrogen fluoride amine salts, alkali metal fluoride salts, and alkaline earth metal fluoride salts.

The amount of fluorine source (A) used is not limited. The lower limit of the amount of fluorine source (A) used can be, for example, 0.1 moles or more, preferably 0.2 moles or more, more preferably 0.3 moles or more, even more preferably 0.4 moles or more, and particularly preferably 0.5 moles or more, per mole of compound (1). The upper limit of the amount of fluorine source (A) used can be, for example, 1000 moles or less, preferably 500 moles or less, more preferably 300 moles or less, even more preferably 100 moles or less, still more preferably 90 moles or less, and particularly preferably 80 moles or less, per mole of compound (1). The amount of fluorine source (A) used can be, for example, within the range of 0.1 to 1000 moles, preferably within the range of 0.2 to 500 moles, more preferably within the range of 0.3 to 100 moles, even more preferably within the range of 0.4 to 90 moles, and particularly preferably within the range of 0.5 to 80 moles.

Fluorine source (A) remaining after the reaction may be trapped and discarded; however, it is preferable to recover and reuse it from the viewpoint of production costs. The trapped residue after the reaction may be washed with, for example, water or alkaline water.

### 2-3. Iodine Source (B)

Of iodine sources (B), the iodoaliphatic compound can be represented, for example, by the formula: R^{C}-I, wherein R^{C} is an alkyl group optionally having one or more substituents.

Preferable examples of R^{C} include C₁-C₁₂ alkanes optionally having one or more substituents. Examples of the substituents include a halogen atom (e.g., an iodine atom). The number of substituents may be within the range of 1 to the maximum substitutable number (e.g., 1, 2, or 3).

Examples of the iodoaliphatic compound include iodomethane, iodoethane, 1-iodopropane, 2-iodopropane, 1-iodobutane, 1-iodo-2-methylpropane, 1-iodopentane, 1-iodo-3-methylbutane, 1-iodohexane, 1-iodoheptane, 1-iodooctane, diiodomethane, 1,8-diiodooctane, and 1,10-diiododecane.

Of iodine sources (B), the iodoaromatic ring compound can be represented, for example, by the formula: R^{D}-I, wherein R^{D} is an aryl group optionally having one or more substituents or a heteroaryl group optionally having one or more substituents.

Preferable examples of R^{D} include a C₆-C₁₈ aryl group optionally having one or more substituents, and a 5- or 6-membered monocyclic aromatic heterocyclic group optionally having one or more substituents. Examples of the substituents include a halogen atom (e.g., a chlorine atom or an iodine atom), an alkyl group (e.g., a C₁-C₄ alkyl group), a fluoroalkyl group (e.g., a fluoro C₁-C₄ alkyl group), R^{E}O- (wherein R^{E} is an alkyl group (e.g., a C₁-C₄ alkyl group)), a carboxyl group, a nitro group, and a cyano group. The number of substituents may be within the range of 1 to the maximum substitutable number (e.g., 1, 2, or 3).

Examples of the iodoaromatic ring compound include iodobenzene, 2-iodotoluene, 3-iodotoluene, 4-iodotoluene, 2,4,6-trimethyliodobenzene, 2-ethyliodobenzene, 3-ethyliodobenzene, 4-ethyliodobenzene, 2-iodoanisole, 3-iodoanisole, 4-iodoanisole, 1-chloro-2-iodobenzene, 1-chloro-3-iodobenzene, 1-chloro-4-iodobenzene, 1,2-diiodobenzene, 1,3-diiodobenzene, 1,4-diiodobenzene, 1-iodo-2-nitrobenzene, 1 iodo-3-nitrobenzene, 1-iodo-4-nitrobenzene, 1-iodo-2-cyanobenzene, 1-iodo-3-cyanobenzene, 1-iodo-4-cyanobenzene, 1-iodo-4-(3,3,4,4,5,5,6,6,7,7,8,8,8-tridecafluorooctyl)benzene, p-iodobenzoic acid, 4-iodobiphenyl, 4,4'-diiodobiphenyl, 4,4"-diiodo-p-terphenyl, 2-iodopyridine, 3-iodopyridine, 4-iodopyridine, 3-iodopyrazole, and 4-iodopyrazole.

Iodine sources (B) may be used singly or in a combination of two or more.

The amount of iodine source (B) used is not limited. The lower limit of the amount of iodine source (B) used can be, for example, 0.01 moles or more, preferably 0.05 moles or more, more preferably 0.1 moles or more, even more preferably 0.15 moles or more, and particularly preferably 0.2 moles or more, per mole of compound (1). The upper limit of the amount of iodine source (B) used can be, for example, 10 moles or less, preferably 9 moles or less, more preferably 8 moles or less, even more preferably 7 moles or less, and still more preferably 6 moles or less, per mole of compound (1). The amount of iodine source (B) used can be, for example, within the range of 0.01 to 10 moles, preferably within the range of 0.05 to 9 moles, more preferably within the range of 0.1 to 8 moles, even more preferably within the range of 0.15 to 7 moles, and still more preferably within the range of 0.2 to 6 moles, per mole of compound (1).

Iodine source (B) remaining after the reaction may be trapped and discarded; however, it is preferable to recover and reuse it from the viewpoint of production costs. The trapped residue after the reaction may be washed with, for example, water or alkaline water.

### 2-4. Compound (C)

In one embodiment, compound (C) is compound (2-1) optionally having one or more substituents. In the "conjugate base of a Brønsted acid" represented by X in formula (2-1), examples of Brønsted acids include inorganic acids and organic acids. Examples of inorganic acids include hydrofluoric acid, hydrochloric acid, perchloric acid, sulfuric acid, persulfuric acid, fluorosulfuric acid, chlorosulfuric acid, phosphoric acid, boric acid, nitric acid, HBF₄, HB₂F₇, HBCl₄, HBCl₃F, HBBr₃F, HPF₆, HAsF₆, HSbF₄, HSbF₆, HSbCl₆, HSbCl₅F, HSb₂F₁₁, HAlF₄, HAlCl₄, HAlCl₃F, and HAlF₃Cl. Examples of organic acids include carboxylic acids, such as acetic acid, trifluoroacetic acid, trichloroacetic acid, and pentafluoropropionic acid; sulfonic acids, such as methanesulfonic acid, trifluoromethanesulfonic acid, trichloromethanesulfonic acid, ethanesulfonic acid, perfluoroethanesulfonic acid, propanesulfonic acid, perfluoropropanesulfonic acid, butanesulfonic acid, perfluorobutanesulfonic acid, benzenesulfonic acid, toluenesulfonic acid, and nitrobenzenesulfonic acid; alkylsulfuric acids, such as methylsulfuric acid; and sulfonyl imide acids, such as bis(methanesulfonyl)imide acid and bis(trifluoromethanesulfonyl)imide acid.

Examples of conjugate bases of the Brønsted acids include ⁻OClO₃, ⁻OSO₂OH, ⁻OSO₂F, ⁻OSO₂Cl, ⁻BF₄, ⁻B₂F₇, ⁻PF₆, ⁻AsF₆, ⁻SbF₆, ⁻AlF₄, ⁻AlCl₄, ⁻SbCl₆, ⁻SbCl₅F, ⁻Sb₂F₁₁, ⁻OSO₂CH₃, ⁻OSO₂CF₃, ⁻OSO₂CCl₃, ⁻OSO₂C₄F₉, ⁻OSO₂C₆H₅, ⁻OSO₂C₆H₄CH₃, ⁻OSO₂C₆H₄NO₂, ⁻OSO₂OCH₃, ⁻N(SO₂CH₃)₂, and ⁻N(SO₂CF₃)₂.

Examples of substituents for compound (2-1) include a halogen atom, a hydroxy group, an alkyl group (e.g., a C₁-C₁₅ alkyl group) optionally having one or more further substituents (e.g., a halogen atom), an alkenyl group (e.g., a C₂-C₁₅ alkenyl group) optionally having one or more further substituents (e.g., a halogen atom), an alkynyl group (e.g., a C₂-C₁₅ alkynyl group) optionally having one or more further substituents (e.g., a halogen atom), an aryl group (e.g., a C₆-C₁₅ aryl group) optionally having one or more further substituents (e.g., a halogen atom, an alkyl group), a nitro group, a cyano group, R^{F}O-, R^{F}CO-, R^{F}SO₂-, R^{F}COO-, (R^{F})(R^{G})NCO-, R^{F}OCO-, R^{F}CONR^{G}-, R^{F}OSO₂-, and (R^{F})(R^{G})NSO₂-, wherein R^{F} and R^{G} are each independently an alkyl group optionally having one or more substituents (e.g., a halogen atom), or an aryl group optionally having one or more substituents (e.g., a halogen atom or an alkyl group). The number of substituents may be within the range of 1 to the maximum substitutable number (e.g., 1, 2, 3, 4, or 5).

In another embodiment, compound (C) is a multimer of compound (2-1) optionally having one or more substituents. An example of the multimer includes a compound represented by the following formula (2-2) optionally having one or more substituents: 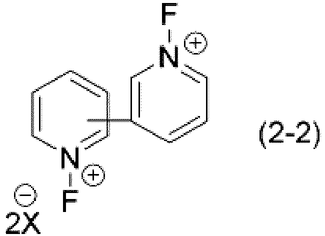 wherein
X is as defined above.

Examples of compound (2-2) optionally having one or more substituents include compounds represented by the following formulas (2-3) to (2-7) optionally having one or more substituents: 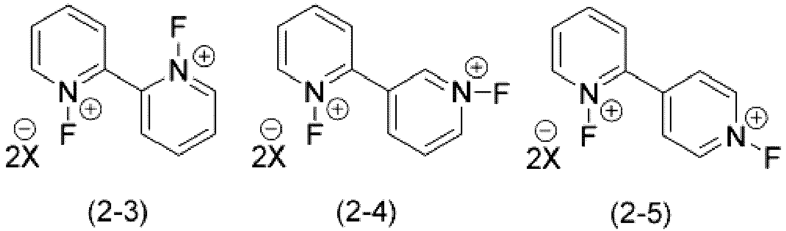 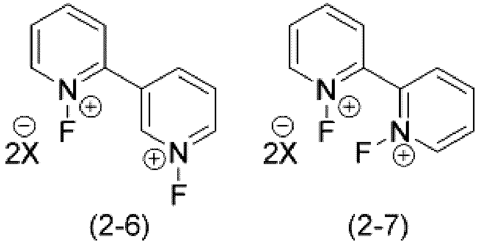 wherein
X is as defined above.
Of these, compound (2-3) optionally having one or more substituents is preferred, and compound (2-3) (not having a substituent) is more preferred.

Examples of substituents for compounds (2-2), (2-3), (2-4), (2-5), (2-6), and (2-7) include the same as the examples of the substituents for compound (2-1). The number of substituents may be within the range of 1 to the maximum substitutable number (e.g., 1, 2, 3, 4, 5, 6, 7, or 8).

Another example of the multimer includes a polymer containing a polymerization unit represented by the following formula (2-8) optionally having one or more substituents: 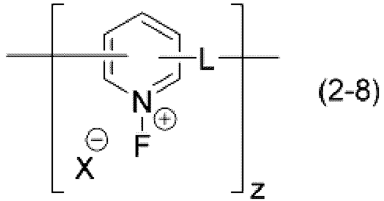 wherein
X is as defined above, L is a single bond, -CH₂-, or -O-, and z is an integer of 2 or more.

In the polymer above, the content of the polymerization unit (2-8) is preferably 50 mol% or more, and more preferably 60 mol% or more.

The polymer may contain a copolymerization unit in addition to the polymerization unit (2-8). Examples of the copolymerization unit include an arylene optionally having one or more substituents (e.g., C₆-C₁₂ arylene, such as phenylene and naphthalenediyl), and a heteroarylene optionally having one or more substituents (e.g., thiophenediyl, furandiyl, pyrrolediyl, pyridinediyl). Examples of the substituent for these copolymerization units include the same as the examples of the substituent for the polymerization unit (2-8).

z is not limited as long as it is an integer of 2 or more, and is preferably 2, 3, or 4. The average molecular weight of the polymer is preferably within the range of 100 to 500000.

Compound (C) may be, for example, an N-fluoropyridinium salt as described in JPH11-040164A.

Compound (C) can be used singly or in a combination of two or more.

The amount of compound (C) used is not limited. The lower limit of the amount of compound (C) used can be, for example, 0.1 moles or more, preferably 0.2 moles or more, more preferably 0.3 moles or more, even more preferably 0.4 moles or more, and particularly preferably 0.5 moles or more, per mole of compound (1). The upper limit of the amount of compound (C) used can be, for example, 10 moles or less, preferably 9 moles or less, more preferably 8 moles or less, even more preferably 7 moles or less, and still more preferably 6 moles or less, per mole of compound (1). The amount of compound (C) used can be, for example, within the range of 0.1 to 10 moles, preferably within the range of 0.2 to 9 moles, more preferably within the range of 0.3 to 8 moles, even more preferably within the range of 0.4 to 7 moles, and still more preferably within the range of 0.5 to 6 moles, per mole of compound (1).

Compound (C) is also preferably used in an amount such that the amount of fluorine on nitrogen in compound (C) is more than 1 mole (e.g., within the range of 1.1 to 3 moles, within the range of 1.2 to 2.5 moles, or within the range of 1.3 to 2 moles) per mole of compound (1).

From the viewpoint of production costs, compound (C) remaining after the reaction is preferably recovered and reused.

Fluorine source (A), iodine source (B), and compound (C) may be added separately to the reaction system or may be added all at once (for example, in the form of a composition described below).

### 2-5. Solvent

The reaction of step A may be performed in the presence or absence of a solvent.

The solvent may be a nonpolar solvent or a polar solvent.

Examples of the solvent include esters, ketones, aromatic hydrocarbons, alcohols, ethers, amines, nitrogen-containing polar organic compounds, nitriles, halogenated hydrocarbons, aliphatic hydrocarbons, fluorine-based solvents, carbonates, other solvents, and combinations thereof.

Examples of esters as the solvent include ethyl acetate, butyl acetate, amyl acetate, ethylene glycol monomethyl ether acetate, and propylene glycol monomethyl ether acetate, and preferably ethyl acetate.

Examples of ketones as the solvent include acetone, methyl ethyl ketone, diethyl ketone, hexanone, methyl isobutyl ketone, heptanone, diisobutyl ketone, acetonylacetone, methylhexanone, acetophenone, cyclohexanone, and diacetone alcohol, and preferably acetone.

Examples of aromatic hydrocarbons as the solvent include benzene, toluene, xylene, and ethylbenzene, and preferably benzene and toluene.

Examples of alcohols as the solvent include methanol, ethanol, n-propanol, isopropanol, n-butanol, pentanol, hexanol, ethylene glycol, diethylene glycol, triethylene glycol, tetraethylene glycol, polyethylene glycol, propylene glycol, dipropylene glycol, tripropylene glycol, polypropylene glycol, trimethylene glycol, and hexanetriol, and preferably methanol and ethanol.

Examples of ethers as the solvent include diethyl ether, dibutyl ether, tetrahydrofuran, tetrahydropyran, dioxane, dimethoxyethane, diethylene glycol diethyl ether, ethylene glycol monomethyl ether, ethylene glycol monoethyl ether, diethylene glycol monomethyl ether, diethylene glycol monoethyl ether, propylene glycol monomethyl ether (PGME; also known as "1-methoxy-2-propanol"), propylene glycol monoethyl ether, triethylene glycol dimethyl ether, triethylene glycol diethyl ether, tetraethylene glycol dimethyl ether, tetraethylene glycol diethyl ether, and anisole, and preferably diethyl ether and tetrahydrofuran.

Examples of amines as the solvent include monoethanolamine, diethanolamine, and triethanolamine.

Examples of nitrogen-containing polar organic compounds as the solvent include N,N-dimethylformamide, N,N-dimethylacetamide, N-methyl-2-pyrrolidone, 2-pyrrolidone, and 1,3-dimethyl-2-imidazolidinone, and preferably N,N-dimethylformamide, N,N-dimethylacetamide, and N-methyl-2-pyrrolidone.

Examples of nitriles as the solvent include acetonitrile, propionitrile, butyronitrile, isobutyronitrile, benzonitrile, and adiponitrile, and preferably acetonitrile.

Examples of halogenated hydrocarbons as the solvent include dichloromethane, dichloroethane, chloroform, carbon tetrachloride, tetrachloroethane, trichloroethane, chlorobenzene, dichlorobenzene, and chlorotoluene, and preferably dichloromethane and chloroform.

Examples of aliphatic hydrocarbons as the solvent include hexane, cyclohexane, heptane, octane, nonane, decane, undecane, dodecane, and mineral spirits, and preferably cyclohexane and heptane.

Examples of fluorine-based solvents include perfluorobenzene, trifluorotoluene, ditrifluorobenzene, and trifluoroethanol, and preferably perfluorobenzene and trifluoroethanol.

Examples of carbonates as the solvent include tetralin dimethyl carbonate, methyl ethyl carbonate, diethyl carbonate, ethylene carbonate, and propylene carbonate, and preferably ethylene carbonate and propylene carbonate.

Examples of other solvents include acetic acid, pyridine, dimethylsulfoxide, sulfolane, and water.

The solvent may be preferably at least one member selected from the group consisting of aliphatic hydrocarbons, aromatic hydrocarbons, halogenated hydrocarbons, and nitriles, more preferably at least one member selected from the group consisting of halogenated hydrocarbons and nitriles, and even more preferably a halogenated hydrocarbon.

These solvents may be used singly, or in a combination of two or more.

The amount of solvent used may be, for example, generally within the range of 0 to 200 parts by mass, preferably within the range of 1 to 100 parts by mass, and more preferably within the range of 5 to 50 parts by mass, per part by mass of compound (1).

Each component may be added to the reaction system of step A at once, in several batches, or continuously.

### 2-6. Temperature and Time

The temperature of step A may be generally within the range of 0 to 200°C, preferably within the range of 10 to 100°C, more preferably within the range of 20 to 100°C, and even more preferably within the range of 40 to 60°C.

The time of step A may be generally within the range of 0.1 to 72 hours, preferably within the range of 0.5 to 48 hours, and more preferably within the range of 1 to 36 hours.

### 2-7. Optional Additional Step B

The method for producing a fluorine adduct of compound (1) may further comprise, in addition to step A, step B of isolating or purifying compound (1). Compound (1) can be isolated or purified by a method such as filtration, extraction, back-extraction, dissolution, concentration, precipitation, dehydration, adsorption, or chromatography, or a combination thereof.

In one embodiment, step B is a step of separating compound (C) and/or a derivative thereof from the reaction mixture obtained in the reaction of step A. The separation step is preferably a step of reducing the reaction mixture with a reducing agent. By such a reduction treatment, compound (C) and/or a derivative thereof can be separated easily and to a high degree.

Examples of the reducing agent include sulfur-based reducing agents, and preferable examples thereof include bisulfites, sulfites, and thiosulfates. Examples of bisulfites include ammonium bisulfite; and alkali metal bisulfites, such as sodium bisulfite and potassium bisulfite. Examples of sulfites include alkali metal sulfites, such as sodium sulfite and potassium sulfite. Examples of thiosulfates include alkali metal thiosulfates, such as sodium thiosulfate and potassium thiosulfate.

The reducing agents may be used singly or in a combination of two or more.

### 3. Composition

The composition of the present disclosure comprises at least one member selected from the group consisting of fluorine source (A) and iodine source (B), and comprises compound (C). Examples of fluorine source (A), iodine source (B), and compound (C) include those similar to those listed above as examples in "2. Method for Producing a Fluorine Adduct of Compound (1)."

Examples of the composition include:
composition α comprising fluorine source (A) and compound (C);
composition β comprising iodine source (B) and compound (C); and
composition γ comprising fluorine source (A), iodine source (B), and compound (C).

In the composition, the amount of fluorine source (A) can be, for example, 0.1 moles or more, 0.2 moles or more, or 0.3 moles or more, and can be 600 moles or less, 500 moles or less, 400 moles or less, 300 moles or less, 200 moles or less, 100 moles or less, 90 moles or less, 80 moles or less, or 70 moles or less, per mole of compound (C). In the composition, fluorine source (A) is contained in an amount, for example, within the range of 0.1 to 600 moles, preferably within the range of 0.2 to 300 moles, and more preferably within the range of 0.3 to 70 moles, per mole of compound (C).

In the composition, the amount of iodine source (B) can be, for example, 0.01 moles or more, 0.05 moles or more, or 0.1 moles or more, and can be 6 moles or less, 5.5 moles or less, or 5 moles or less, per mole of compound (C). In the composition, iodine source (B) is contained in an amount, for example, within the range of 0.01 to 6 moles, preferably within the range of 0.05 to 6 moles, and more preferably within the range of 0.1 to 5 moles, per mole of compound (C).

The composition can be preferably used as a fluorinating agent (in particular, a fluorinating agent for a compound having a carbon-carbon double bond or triple bond, such as compound (1)). Thus, the present disclosure encompasses a fluorinating agent comprising the composition described above (in particular, a fluorinating agent for a compound having a carbon-carbon double bond or triple bond, such as compound (1)).

### Examples

One embodiment of the present disclosure is described in more detail below with reference to Examples; however, the present disclosure is not limited thereto.

### Example 1

Triethylamine (TEA)•5HF (1.25 mL) and 1,1'-difluoro-2,2'-bipyridinium bis(tetrafluoroborate) (0.75 mmol) were added to a solution of 1-dodecene (0.5 mmol) and 4-iodotoluene (0.1 mmol) in dichloroethane (DCE) (2 mL), and the mixture was allowed to react at 40°C for 22 hours. The reaction mixture was poured into saturated sodium hydrogen carbonate, followed by the addition of sodium sulfite. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Methyl 4-fluorobenzoate was added as an internal standard, and the yield of 1,2-difluorododecane was analyzed by ¹⁹F NMR to be 68%.

The aqueous layer was made sufficiently alkaline with 2M NaOH and then extracted 3 times with ethyl acetate (20 mL). After washing with a saturated aqueous sodium chloride solution, the resulting product was dried over anhydrous sodium sulfate, the solvent was removed using an evaporator, and drying was performed in a desiccator under reduced pressure. 2,2'-Bipyridyl was recovered in a yield of 84%.

### Example 2

Pyridine (Py)•9HF (0.52 mL, 20 mmol HF) and 1,1'-difluoro-2,2'-bipyridinium bis(tetrafluoroborate) (1.2 mmol) were added to a solution of ethyl cinnamate (1.0 mmol) and 4-iodotoluene (0.2 mmol) in dichloromethane (DCM) (5 mL), and the mixture was allowed to react at 40°C for 24 hours. The reaction mixture was poured into saturated sodium hydrogen carbonate, followed by the addition of an aqueous sodium thiosulfate solution. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Fluorobenzene was added as an internal standard, and the yield of the target product, i.e., ethyl 3,3-difluoro-2-phenylpropanoate, was analyzed by ¹⁹F NMR to be 50%.

The aqueous layer was made sufficiently alkaline with 2M NaOH and then extracted with ethyl acetate. After washing with a saturated aqueous sodium chloride solution, the resulting product was dried over anhydrous sodium sulfate, the solvent was removed using an evaporator, and drying was performed in a desiccator under reduced pressure. 2,2'-Bipyridyl was recovered in a yield of 49%.

### Example 3

Triethylamine (TEA)•5HF (2.5 mL) and 1,1'-difluoro-2,2'-bipyridinium bis(tetrafluoroborate) (1.5 mmol) were added to a solution of 1-octene (1 mmol) and 4-iodotoluene (0.2 mmol) in dichloromethane (DCM) (5 mL), and the mixture was allowed to react at 40°C for 22 hours. The reaction mixture was poured into saturated sodium hydrogen carbonate, followed by the addition of sodium sulfite. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Methyl 4-fluorobenzoate was added as an internal standard, and the yield of 1,2-difluorooctane was analyzed by ¹⁹F NMR to be 54%.

### Example 4

The same procedure as in Example 3 was performed except that 1-octene was changed to 10-undecen-1-ol, thereby obtaining 10,11-difluoroundecan-1-ol in a yield of 62%.

### Example 5

The same procedure as in Example 3 was performed except that 1-octene was changed to 5-bromo-1-pentene, thereby obtaining 5-bromo-1,2-difluoropentane in a yield of 73%.

### Example 6

Triethylamine (TEA)•5HF (1 mL) and 1,1'-difluoro-2,2'-bipyridinium bis(tetrafluoroborate) (0.5 mmol) were added to a solution of methylacetate-1-nonene (0.4 mmol) and 4-iodotoluene (0.08 mmol) in dichloroethane (DCE) (2 mL), and the mixture was allowed to react at 40°C for 22 hours. The reaction mixture was poured into saturated sodium hydrogen carbonate, followed by the addition of sodium sulfite. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Methyl 4-fluorobenzoate was added as an internal standard, and the yield of methylacetate-1,2-difluorononane was analyzed by ¹⁹F NMR to be 67%.

### Example 7

The same procedure as in Example 6 was performed except that methylacetate-1-nonene was changed to allylbenzene, thereby obtaining 2,3-difluoropropylbenzene in a yield of 85%.

### Example 8

Triethylamine (TEA)•5HF (1.25 mL) and 1,1'-difluoro-2,2'-bipyridinium bis(tetrafluoroborate) (0.63 mmol) were added to a solution of 1-allyl-2-methylbenzene (0.5 mmol) and 4-iodotoluene (0.1 mmol) in dichloromethane (DCM) (3 mL), and the mixture was allowed to react at 40°C for 22 hours. The reaction mixture was poured into saturated sodium hydrogen carbonate, followed by the addition of sodium sulfite. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Methyl 4-fluorobenzoate was added as an internal standard, and the yield of 1-(2,3-difluoropropyl)-2-methylbenzene was analyzed by ¹⁹F NMR to be 100%.

### Example 9

Pyridine (Py)•9HF (0.26 mL, 10 mmol HF) and 1,1'-difluoro-2,2'-bipyridinium bis(tetrafluoroborate) (1.2 mmol) were added to a solution of ethyl 3-(4-bromophenyl)acrylate (0.5 mmol) and 4-iodotoluene (0.1 mmol) in dichloromethane (DCM) (3 mL), and the mixture was allowed to react at 40°C for 20 hours. The reaction mixture was poured into saturated sodium hydrogen carbonate, followed by the addition of an aqueous sodium thiosulfate solution. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Fluorobenzene was added as an internal standard, and the yield of the target product, i.e., ethyl 2-(4-bromophenyl)-3,3-difluoropropanoate, was analyzed by ¹⁹F NMR to be 95%.

### Example 10

Triethylamine (TEA)•5HF (1.5 mL) and 1,1'-difluoro-2,2'-bipyridinium bis(tetrafluoroborate) (0.6 mmol) were added to a solution of chalcone (0.4 mmol) and 4-iodotoluene (0.08 mmol) in dichloroethane (DCE) (1.5 mL), and the mixture was allowed to react at 50°C for 22 hours. The reaction mixture was poured into saturated sodium hydrogen carbonate, followed by the addition of an aqueous sodium thiosulfate solution. 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Fluorobenzene was added as an internal standard, and the yield of the target product, i.e., 3,3-difluoro-1,2-diphenyl-1-propanone, was analyzed by ¹⁹F NMR to be 91%.

### Example 11

The same procedure as in Example 10 was performed except that chalcone was changed to 3-(4-fluorophenyl)-1-phenyl-2-propen-1-one, thereby obtaining 3,3-difluoro-2-(4-fluorophenyl)-1-phenylpropan-1-one in a yield of 95%.

### Example 12

The same procedure as in Example 10 was performed except that chalcone was changed to 3-(4-fluorophenyl)-1-tolyl-2-propen-1-one, thereby obtaining 3,3-difluoro-2-(4-fluorophenyl)-1-tolylpropan-1-one in a yield of 92%.

### Example 13

The same procedure as in Example 10 was performed except that chalcone was changed to 3-phenyl-1-(p-tolyl)-2-propen-1-one, thereby obtaining 3,3-difluoro-2-phenyl-1-(p-tolyl)propan-1-one in a yield of 85%.

### Example 14

The same procedure as in Example 10 was performed except that chalcone was changed to 1-(4-chlorophenyl)-3-phenyl-2-propen-1-one, thereby obtaining 1-(4-chlorophenyl)-3,3-difluoro-2-phenylpropan-1-one in a yield of 87%.

### Example 15

The same procedure as in Example 10 was performed except that chalcone was changed to 1-(4-fluorophenyl)-3-phenyl-2-propen-1-one, thereby obtaining 1-(4-fluorophenyl)-3,3-difluoro-2-phenylpropan-1-one in a yield of 94%.

### Example 16

The same procedure as in Example 10 was performed except that chalcone was changed to 4-phenyl-3-buten-2-one, thereby obtaining 4,4-difluoro-3-phenylbutan-2-one in a yield of 47%.

### Comparative Example 1

TEA•5HF (1.0 mL) and Selectfluor (trademark) (0.48 mmol) were added to a solution of 1-dodecene (0.4 mmol) and 4-iodotoluene (0.08 mmol) in DCE (2 mL), and the mixture was allowed to react at 40°C for 21 hours. The reaction mixture was poured into saturated sodium hydrogen carbonate, followed by the addition of sodium sulfite (1 g). 2M NaOH was added and extracted with ethyl acetate. The ethyl acetate extract was then washed with 2M HCl. The organic layer was washed with a saturated aqueous sodium chloride solution and dried over anhydrous sodium sulfate, and the solvent was removed using an evaporator. Methyl 4-fluorobenzoate was added as an internal standard, and the yield of 1,2-difluorododecane was analyzed by ¹⁹F NMR to be 80%.

The aqueous layer was made sufficiently alkaline with 2M NaOH and then extracted with ethyl acetate. In the extracted ethyl acetate, Selectfluor (trademark) or a derivative thereof was not at all observed based on H-NMR analysis. This experiment demonstrated that recovering Selectfluor (trademark) by organic solvent extraction is difficult.

## Claims

1. A method for producing a fluorine adduct of a compound represented by the following formula (1): 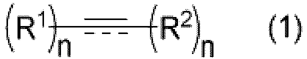 wherein
R¹ and R² are each independently a hydrogen atom or an organic group, or R¹ and R² optionally form a ring together with the two adjacent carbon atoms;
n is 1 or 2; and
the symbol 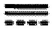 is a double bond or a triple bond,
with the proviso that
when the symbol is a triple bond, n is 1,
when the symbol is a double bond, n is 2,
two R¹s are optionally the same or different,
two R²s are optionally the same or different, or
two R¹s or two R²s optionally form a ring together with their adjacent carbon atom,
the method comprising reacting the compound represented by formula (1) with
(A) at least one fluorine source selected from the group consisting of hydrogen fluoride, hydrogen fluoride salts, and fluoride salts;
(B) at least one iodine source selected from the group consisting of iodine, iodoaliphatic compounds, and iodoaromatic ring compounds; and
(C) at least one compound selected from the group consisting of compounds represented by the following formula (2-1) optionally having one or more substituents, and multimers thereof: 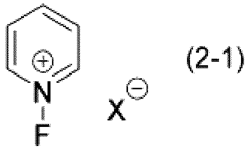 wherein
X is a conjugate base of a Brønsted acid,
to add fluorine to the double bond or triple bond.

2. The production method according to claim 1, wherein the compound (C) is a compound represented by the following formula (2-2) optionally having one or more substituents: 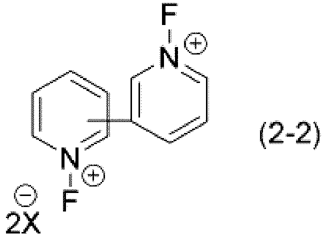 wherein
X is as defined above.

3. The production method according to claim 1, wherein the compound (C) is a compound represented by the following formula (2-3) optionally having one or more substituents: 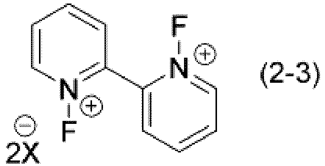 wherein
X is as defined above.

4. The production method according to claim 3, wherein the compound (C) is a compound represented by formula (2-3).

5. The production method according to any one of claims 1 to 4, wherein the compound (C) is used in an amount within the range of 0.1 to 10 moles per mole of the compound represented by formula (1).

6. The production method according to any one of claims 1 to 4, wherein the compound (C) is used in an amount such that the amount of fluorine on nitrogen in the compound (C) is more than 1 mole per mole of the compound represented by formula (1).

7. The production method according to any one of claims 1 to 4, wherein the fluorine source (A) is at least one member selected from the group consisting of hydrogen fluoride, hydrogen fluoride amine salts, alkali metal fluoride salts, and alkaline earth metal fluoride salts.

8. The production method according to any one of claims 1 to 4, wherein the fluorine source (A) is used is an amount within the range of 0.1 to 1000 moles per mole of the compound represented by formula (1).

9. The production method according to any one of claims 1 to 4, wherein the iodine source (B) is used in an amount within the range of 0.1 to 10 moles per mole of the compound represented by formula (1).

10. The production method according to any one of claims 1 to 4, wherein the reaction is performed in the presence of a solvent.

11. The production method according to any one of claims 1 to 4, wherein R¹ and R² are each independently a hydrogen atom, an alkyl group optionally having one or more substituents, a cycloalkyl group optionally having one or more substituents, an aryl group optionally having one or more substituents, an aralkyl group optionally having one or more substituents, a non-aromatic heterocyclic group optionally having one or more substituents, a cyano group, a formyl group, R^{A}O-, R^{A}CO-, R^{A}SO₂-, R^{A}COO-, (R^{A})(R^{B})NCO-, R^{A}OCO-, R^{A}CONR^{B}-, R^{A}OSO₂-, or (R^{A})(R^{B})NSO₂-, wherein R^{A} and R^{B} are each independently an alkyl group optionally having one or more substituents, an aryl group optionally having one or more substituents, or an aralkyl group optionally having one or more substituents; or
R¹ and R² form a ring together with the two adjacent carbon atoms.

12. The production method according to any one of claims 1 to 4, further comprising separating the compound (C) and/or a derivative thereof from a reaction mixture after the reaction.

13. The production method according to any one of claims 1 to 4, further comprising reducing a reaction mixture after the reaction with a reducing agent.

14. The production method according to claim 13, wherein the reducing agent is a sulfur-based reducing agent.

15. A composition comprising at least one member selected from the group consisting of the following fluorine source (A) and iodine source (B), and comprising the following compound (C):
(A) at least one fluorine source selected from the group consisting of hydrogen fluoride, hydrogen fluoride salts, and fluoride salts;
(B) at least one iodine source selected from the group consisting of iodine, iodoaliphatic compounds, and iodoaromatic ring compounds; and
(C) at least one compound selected from the group consisting of compounds represented by the following formula (2-1) optionally having one or more substituents, and multimers thereof: 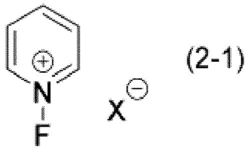 wherein
X is a conjugate base of a Brønsted acid.

16. A fluorinating agent comprising the composition according to claim 15.
